Europäisches Patentamt

(19) European Patent Office

Office européen des brevets

(11) Publication number: **0 077 571**
A2

# (12) EUROPEAN PATENT APPLICATION

(21) Application number: 82109658.3

(22) Date of filing: 19.10.82

(51) Int. Cl.³: **C 12 F 1/00**
C 12 N 15/00, C 12 P 21/00
//C12R1/91, C12N5/00

(30) Priority: 19.10.81 JP 166559/81
19.10.81 JP 166560/81

(43) Date of publication of application:
27.04.83 Bulletin 83/17

(84) Designated Contracting States:
CH DE FR GB LI SE

(71) Applicant: AJINOMOTO CO., INC.
5-8, Kyobashi 1-chome, Chuo-ku
Tokyo 104(JP)

(71) Applicant: Hamaoka, Toshiyuki
1022, Komuinshukusha, 5-730
Gakuendaiwa-cho Nara-shi(JP)

(72) Inventor: Hamaoka, Toshiyuki
No. 1022, Komuinshukusha, 5-730
Gakuendaiwa-cho Nara-shi(JP)

(72) Inventor: Takatsu, Kiyoshi
No. 941, Komuinshukusha, 5-730
Gakuendaiwa-cho Nara-shi(JP)

(72) Inventor: Yoshimoto, Ryota
No. 958, Kashimada, Saiwai-ku
Kawasaki-shi Kanagawa-ken(JP)

(72) Inventor: Hamuro, Junji
No. 241-32, Fukaya-cho, Totsuka-ku
Yokohama-shi Kanagawa-ken(JP)

(74) Representative: Schübel-Hopf, Ursula et al,
Strehl, Schübel-Hopf, Schulz Patentanwälte
Widenmayerstrasse 17
D-8000 München 22(DE)

(54) Process for producing a lymphokine.

(57) A lymphokine in purified form or in the form of a lymphokine containing culture supernatant may be obtained by collecting lymphocytes from an animal or a human sensitized by an immunogen which induces the production of an antibody, selectively concentrating the lymphokine producing cells, forming a fused cell hybrid between the lymphokine producing cells and animal derived tumor cells, cloning the obtained hybrid, selecting the clone which spontaneously produces the lymphokine, culturing the clone to produce the lymphokine, and optionally recovering the lymphokine from the culture and purifying the lymphokine. This process is especially effective for producing interleukin 2, colony stimulating factor, T cells replacing factor, macrophage activating factor or immune interferon.

EP 0 077 571 A2

# PROCESS FOR PRODUCING A LYMPHOKINE

This invention relates to the production of immunologically active substances with living cells, and more specifically to the production of lymphokines by employing the cell fusion process.

When T lymphocytes are stimulated and activated by an antigen or mitogen, a variety of lymphokines, which are proteinous biologically active soluble factors, are released into the medium.

. Examples of these lymphokines include immune interferon (hereinafter sometimes referred to as immune IFN), colony stimulating factor ( hereinafter sometimes referred to as CSF), T lymphocyte replacing factor (hereinafter sometimes referred to as TRF), macrophage activating factor (hereinafter sometimes referred to as MAF), interleukin 2 (hereinafter sometimes referred to as IL 2) etc. These lymphokines are all produced from T lymphocytes and exhibit immunological activity. Owing to their immunological activities and biological activities these lymphokines are widely useful against diseases caused by immunological deficiency or abnormality and are also strikingly useful for curing a vital body from a disease or functional abnormality by the artificial control of cellular immunity and humoral immunity. Further, they provide an important method for diagnosing the immunological function useful for the clinical diagnosis of immunological diseases due to abnormal production and response of lymphokines and are also useful as reagents for researches in the whole area of biology and medicine.

Heretofore, lymphokines have been prefered by sensitizing a mouse using an antigen, removing the spleen cells and resensitizing them in vitro using the same antigen to produce lymphokines in the medium, or by stimulating lymphocytes of various sources using a mitogen, i.e. a polyclonal activator for lymphocytes. However, in the above-described methods which stimulate the spleen cells of a normal mouse using an antigen, it is necessary to collect large amounts of lymphocytes from the vital body in order to produce even small amounts of lymphokines, and moreover, these lymphocytes can hardly survive if the culturing time lasts more than a week. Thus these methods are not useful for the continuous pro- duction by a long-term successive culture.

In order to solve a part of the above-described problems, attempts to prepare a mouse lymphokine using fused cells obtained by fusing T lymphocytes and mouse thymoma cells have been made recently (J. Exp. Med., 148, 373 (1978); Nature, 267, 707 (1977); Eur. J. Immunol., 7, 707 (1977); J. Immunol., 121, 2113 (1978); Nature, 283, 197 (1980); and Nature, 291, 502 (1981)). However, most of the thus obtained hybridomas do not exist any longer because of their instability of the lymphokine productivity and of the proliferating ability. Further most of them required the addition of a stimulant for the production of lymphokines, however, under the used conditions, the hybridomas are not able to survive during the lymphokine production in the presence of a stimulant. Thus their continuous use for the production of lymphokines by the long-term continuous culture is not possible.

It has recently been described in J. Immunol., 124, 2414 (1980) that one of the lymphokines, T lymphocyte replacing factor (TRF), may be produced by in vitro restimulation of T lymphocytes, which had been immunized with Mycobacterium tuberculosis, with purified protein (PPD) of Mycobacterium tuberculosis, but such TRF producing cells are not capable of being cultured by a long-term continuous culture and thus it has heretofore been impossible to produce TRF applicable in practical uses. On the other hand, in the process by Takatsu et al., since the stimulation with PPD was required, the factor thus obtained was not free from PPD and its removal was difficult.

It is therefore an object of the invention to provide a process which is applicable for the continuous mass production of lymphokine, which is based on fusing lymphokine producing cells with thymoma cells or other tumor cells, cloning of cell lines which may be proliferated in vitro and may spontaneously and continuously produce lymphokines without the need of stimulation by PPD. Such process accordingly should allow the application of the lymphokines to patients suffering from diseases caused by abnormality of the antibody production function and in cellular immunity function due to disorders of immunological functions.

According to the invention there is provided a process for producing a lymphokine in purified form or as a lymphokine containing culture supernatant, which is characterized by the following process steps :

(1)   collecting lymphocytes from an animal or a human sensitized by an immunogen which induces the production of an antibody,

(2)   selectively concentrating the lymphokine producing cells,

(3)   forming a  fused cell hybrid between the lymphokine producing cells and animal derived tumor cells,

(4)   cloning the obtained hybrid,

(5)   selecting the clone which spontaneously produces the lymphokine,

(6)   culturing the clone to produce the lymphokine, and optionally

(7)   recovering the lymphokine from the culture and purifying the lymphokine.

Recently, in J. Immunol., 125, 2646-2653 (1980) the present inventors have disclosed a method for establishing a fused cell line which spontaneously and continuously produces T lymphocyte replacing factor, one kind of lymphokine, by sensitizing the spleen cells, obtained from a mouse sensitized with tuberculin, with a protein component isolated from tuberculin in vitro, and thereafter fusing them with mouse thymoma cells BW 5147. Such a fused cell line which spontaneously and continuously produces a lymphokine has allowed the continuous production of lymphokines by the long-term successive culture, and has given the possibility of the lymphokine production in a commercial scale. The used process for preparing a hybridoma producing lymphokines spontaneously had never been known till then.

However, when the process and cell line disclosed by the present inventors in the aforesaid journal were employed, it was found that there was a grave disadvantage that although the fused cells proliferate and a lymphokine is also produced when cultured using a Petri dish of 10 cm or smaller in diameter and a small amount, such as 10 ml of a culture medium, the hybridoma does not proliferate at all after the start of culture in a greater scale, and accordingly the TRF is not produced or accumulated.

Under such circumstances, the present inventors have been intensively studying for the purpose of developing and establishing a process for producing lymphokines by culturing a hybridoma which continuously produces a lymphokine and thus may be employed industrially by carrying out a long-term successive culture of the aforesaid T lymphocyte-derived hybridoma which spontaneously produces a useful lymphokine. As a result, it was found that even the T lymphocyte-derived hybridoma whose mass-production has been difficult unlike the cases of many cell lines and B lymphocyte-derived hybridomas can proliferate in a great amount and efficiently produce a great amount of the lymphokine, if the culture conditions are improved appropriately. The lymphokine of this invention may be recovered from the culture supernatant after culturing a hybridoma by purifying operations, but if necessary, the whole culture may also be employed depending on the purpose.

For preparing an antibody against the lymphokine, an animal different from that from which the lymphokine producing cells have been derived is immunized with the lymphokine, and B cells are collected, after which the B cells are fused with myeloma cells in conventional manner well known in this technical field to form a hybridoma which produces a monoclonal antibody, thereby said antibody may be easily obtained. For example, see the process disclosed in Proc. Nat. Acd. Sci. U.S.A., 74, 2985 (1977). The antibody against the lymphokine is important as a clinical diagnostic agent e.g. for the measurement of the lymphokine level in blood etc.

The somatic cell hybridoma of this invention may be produced by fusing thymoma cells and lymphokine producing cells.

Preferably employed as lymphokine producing cells are spleen cells or lymph node cells of an animal immunized with Mycobacterium tuberculosis, cells obtained from tuberculin-positive human peripheral blood, or cells or human peripheral blood lymphocytes activated in vitro with an immunogen such as PPD etc. The specific species of animals, from which the thymoma cells and the lymphokine producing cells are derived, may be different from each other as long as cells of one species are capable of being fused with cells of other species. In other words, such pairs as mouse to rat, rat to human or mouse to human may be employed. However, it is more advantageous for the stability of the proliferating activity of the resultant hybridoma to employ animals belonging to the same species (including humans) as sources for both thymoma cells and lymphokine producing cells.

Methods for isolation and concentrating T lymphocytes from the spleen, lymph nodes or peripheral blood are known in literature. However, it has not been known before this invention whether lymphokine producing cells can be concentrated according to the method for concentrating T lymphocytes.

In accordance with this invention, B lymphocytes may be removed by treating with antiimmunoglobulin antiserum and complement, and phagocytes may be removed by removing the cells adsorbed in a Sephadex (gel filterating agent produced by Pharmacia Fine Chemicals Co.) column. When a nylon fiber column is employed, both adhering B lymphocytes and phagocytes may be simultaneously removed. Further, if the animal which provides the lymphokine producing cells is treated by X-ray or by administration of cyclophosphamide, the suppressor T lymphocytes are killed and thus the concentration of the lymphokine producing cells may be more efficiently effected.

One preferred cell line for the practice of this invention is a fused cell hybrid between Mycobacterium tuberculosis-sensitized mouse spleen cells and mouse thymoma cells. By using the somatic cell hybrid between the lymphokine producing cells from the Mycobacterium tuberculosis-immunized BALB/C mouse and the thymoma cells of an AKR mouse excellent results were obtained. Especially preferred mouse thymoma cells are called BW 5147 (easily available among those skilled in the art). The cell fusion itself may be effected in conventional manner.

After the cells have been fused, each hybrid clone grows from each hybridoma and lymphokine producing hybrid clones are selected. The clone selection may be easily effected by techniques well known in this field. For example, see J. Exp. Med., 150, 1510 (1970) for IL 2, and J. Cell Comp. Physiol., 66, 319 (1965) for CSF.

According to this invention it is important that the lymphokine producing hybridoma, which spontaneously produces a lymphokine without adding PPD may be selected independently from PPD addition.

The selected lymphokine producing hybridoma may be maintained in a long-term continuous culture in large amounts in vitro, or may be grown in a histo-compatible animal or a thymus-deficient nude mouse. The hybridoma may be transplanted or injected into a host animal. The hybridoma may be recovered from the medium, or from the serum or from ascites of the animal by means well known in the art. For example, see Proc. Nat. Acd. Sci. U.S.A., 75, 1510 (1978).

One typical process for producing a cell line of hybridoma is now described below. While this process is explained with respect to the fusion of thymoma cells of an AKR mouse and spleen cells of an immunized BALB/C mouse, it is to be understood that this process may also be applied to the production using other tumor cells and T cells which produce other lymphokines. Similarly, while this process immunizes the animal by Mycobacterium tuberculosis, also other immunogens may be employed.

(a) Obtaining Lymphokine Producing Cells for Fusion

A BALB/C mouse is immunized with 500 μg of mycobacterium tuberculosis in a conventional manner well known in the art. Three weeks after the immunization, the animal is killed, and the spleen cells and lymph node cells are collected. A cell suspension is prepared according to the method taught in Eur. J. Immunol., 5, 720 (1975). The spleen cells and lymph node cells are mixed at a ratio of 1 : 1, and treated with goat antimouse immature thymus B lymphocyte antibody and complement to remove the immature thymus gland cells and B lymphocytes. The treated mixture is then passed through a Sephadex G-10 column to remove the dead cells and phagocytes.

(b)  Producing Thymoma Cells for Fusion

Thymoma cell strain BW 5147.AZG$^R$.OU$^R$ derived from an AKR mouse deficient in hypoxanthine phosphoribosyl transferase is maintained in a conventional medium. The growth of the BW 5147.AZG$^R$.OU$^R$ is inhibited by a mediun containing hypoxanthine aminopterin and thymidine.

It is more preferred to incorporate about 3 x 10$^{-6}$ mol

of glycine into said medium.

(c) Producing the Hybridoma

Lymphokine producing cells and thymoma cells are fused in the presence of polyethylene glycol (PEG) 1000 as described in e.g. Eur. J. Immunol., 6, 511 (1976) and Somat. Cell. Genet.,

3, 231 (1977).  Although Sendai virus may be employed instead of PEG,

the use of latter  is simpler.  After the fusion, the cells are suspended in the hypoxanthine aminopterin thymidine selective medium described in Science, 115, 709 (1969), and inoculated into a flask or into respective wells on a tissue culture plate.

Thereafter by a test it is determined whether a given clone of hybridoma actually produces a lymphokine or not. The method therefor is illustrated in the examples described hereinafter.

The thus obtained lymphokine producing T lymphocyte hybrid may be cultured in a scale of 10 - 20 ml in a conventional tissue culture medium using a Petri dish or a flask. When mass-culture is required, a shaking culture,rotatory culture, stirred culture etc.

may be carried out with or without aeration by air containing $CO_2$ gas. However, a mass-culture is not possible with an ordinary static culture.

It has been discovered that T lymphocyte-derived hybridoma generally has a higher oxygen demand compared with normal cells, and that the culture in a larger scale is difficult because it causes the deficiency of oxygen dissolved in the medium and thus the proliferation of the cells is suppressed. For example, when the T-lymphocyte-derived hybridoma which spontaneously produces TRF, i.e. B151-K12 disclosed by the present inventors in J. Immunol., 125, 2646-2653 (1980) (which may be easily prepared by those skilled in the art according to the teachings in said publication) is statically cultured using a Petri dish or a flask, the cells do not proliferate at all if the thickness of the medium (the distance from the bottom of the flask to the liquid surface of the medium) exceeds 3mm and accordingly no TRF is produced in the medium. Therefore, if such static culture is carried out, only the production of TRF in a scale of about 50 ml at most is possible.

Regarding these facts the present inventors have discovered that a process for the mass-production of a T lymphocyte-derived hybridoma may be carried out successfully, if an adequate amount of oxygen is supplied to the cells by enhancing the amount of oxygen dissolved in the medium. This may be achieved by employing the following three roughly classified modes, which are part of the present invention.

(a)  Shaking Culture

One mode accomplished by this invention is a method which carries out the culture by using e.g. a flask and shaking it instead of allowing it to stand. According to this invention, by shaking the flask at an appropriate inclination (15-30°C) in such way that it is shaken right and left like a seesaw at a rate of 2 - 10 times per minute, a method has been established in which the hybridoma stably proliferates even though the thickness of the medium is 3 mm or more, and in which the cells of this hybridoma can spontaneously produce a lymphokine, said lymphokine is also produced quite similarly to the case where the hybridoma is cultured in a static flask under the optimum conditions in a small scale. In such case, the flask to be used for the culture may be any for conventional cell culture, and also the method of shaking is not limited to the seesaw-like movement, but any which can sufficiently agitate the medium may be employed.

For example, in the case of B151-K12, when the thickness of the medium exceeds 3 mm, the hybridoma does not proliferate at all and thus no TRF is produced in the static culture, but by practicing this invention, it is possible to proliferate the hybridoma and produce TRF even when the liquid depth is 10 - 30 mm.

(b)  Rotatory Culture

The present inventors have also achieved a method which comprises culturing a T lymphocyte-derived hybridoma using a roller bottle (rotatory incubator) to produce a lymphokine in the medium.  By rotating the roller bottle and maintaining the inside of the roller bottle under     regularly agitated conditions, the cells uniformly float throughout the medium and oxygen is continuously      dissolved into the medium from the gas phase, and thus the deficiency of the amount of dissolved oxygen is replenished.

For example, when B151-K12 is cultured in a medium of 6 1 in a roller bottle of a capacity of 10 1, by rotating the roller bottle at a rate of 9 - 20 rpm, the cells, at an initial concentration of $0.2 \times 10^5$ cell/ml, proliferate 5- to 10-fold in 3 - 4 days and thus the proliferation comparable to the static culture under the optimum conditions in small scale may be achieved. Further, in this case, TRF is produced from B151-K12 in a given volume of the medium in an amount comparable to that obtained in the static culture under the optimum conditions in small scale.

(c)  Stirred Culture

A further novel method for culturing a T lymphocyte-derived hybridoma accomplished by this invention is using a cell culture flask or cell culture tank equipped with a stirrer for the medium and stirring the medium to eliminate the problem of the deficiency of the amount of oxygen dissolved in the medium. Where this invention is practiced in a culture tank, the deficiency of the dissolved oxygen may also be replenished by culturing the hybridoma by aerating $O_2$ containing air. By carrying out this invention using the T lymphocyte-derived hybridoma which spontaneously produces a lymphokine, the mass-production of the T lymphocyte-derived lymphokine may be easily effected.

For example, in the case of B151-K12, by establishing the initial cell concentration at $0.2 \times 10^5$ cells/ml and using 1 - 5 l of medium the number of the cells reaches 10 - 20 times the initial concentration in 3 - 4 days. At the same time, TRF is produced in the medium.

The procedures for producing lymphokines from the T lymphocyte-derived hybridoma by employing either of the above described (a), (b) and (c) are outlined below.

In has been found that once the T lymphocyte-derived hybridoma which produces a lymphokine has been obtained, it may be cultured in a large scale by employing any of (a), (b) and (c) or a combination thereof and other conditions which generally permit the supply of adequate oxygen to the cells by enhancing the amount of oxygen dissolved in the medium.

When the present invention is carried out using the method described above as (a), (b), (c) etc., the medium may be an ordinary medium used for culture of mammalian cells. Representative examples are RPMI-1640, Creck medium, Dulbecco modified Eagle medium etc.  It is preferred to add 20 % or less of the serum of a mammal to the medium,          although culture in the absence of serum may be conducted by adding serum proteins such as albumin, or other nutrients, instead of the serum. Further, while culture is preferably conducted in air containing 5 - 10% of $CO_2$ by slightly opening the stopper of the incubator so as to maintain the pH of the medium constant, culture under the closed conditions with the stopper of the incubator closed may also be effected. When the culture is conducted using a   culture tank, it is preferred to culture by aerating with air containing $O_2$ and $CO_2$ gases.

When the T lymphocyte hybridoma requires a stimulant such as phytohemmagglutinin, concanavalin A, pokeweed mitogen, protein A, bacterial cells and components derived therefrom etc. for the production of the lymphokine, the stimulant may be added to the medium when the proliferation of the cells has reached the predetermined cell concentration.  Further, the production of the lymphokine from the hybridoma may be enhanced by adding a phorbol ester, hydroxyurea, interleukin 1 etc., as required, at an appropriate concentration to the medium.

Recovery and purification of the lymphokine from the hybridoma culture supernatant may be effected by appropriately employing various methods such as salting out, concentration, vacuum dialysis, gel permeation chromatography, ion exchange chromatography, preparatory isoelectric electrophoresis, gel electrophoresis etc. either alone or in combination.

The following examples illustrate the embodiments of the present invention, but it should be understood that they are a mere illustration of the present invention and should in no way restrict the scope of the invention.

Example 1

Preparation of T Lymphocyte Hybridoma Which Spontaneously Produces TRF and Mass-production of TRF by Its Mass-culture

(a)   Production of Hybridoma

Spleen cells and lymph node cells were removed from a BALB/C mouse (commercially available mouse immunized with 500 μg of Mycobacterium tuberculosis human type Aoyama B, and suspended in a medium by the method disclosed in Eur. J. Immunol., 5, 720 (1975), and treated with the antibody and complement to the B cells and immature thymus gland cells by the method disclosed in Cellular Immunol., 23, 140 (1976) to remove these cells. Further, the cell suspension was passed through a Sephadex G-10 (produced by Pharmacia Fine Chemicals Co.) column to remove the phagocytes and dead cells to prepare a cell suspension having an increased concentration of the T cells.

This was then fused with thymoma cells derived from an AKR mouse BW5147·AZG$^R$·OU$^R$ (commercially available mouse deprived of hypoxanthine phosphoribosyl transferase using polyethylene glycol (PEG) 1000. Ten to twenty days after the fusion, a hybridoma grown in a hypoxanthine·aminopterin·thymidine (HAT) selective medium appeared, and it was cultured in a hypoxanthin· thymidine (HT) medium for further 3 - 7 days. Thus, 170 kinds of hybridoma clones were obtained.

(b)  Selection of TRF Producing Hybridoma Clone

The obtained hybridomas were each cultured at an initial concentration of $0.2 \times 10^5$ cells/ml in a scale of 5 ml/flask (6 cm in diameter) using a 10% fetal bovine serum containing RPMI-1640 medium for 3 - 4 days, and each culture supernatant was collected.  This culture supernatant was tested to judge whether TRF had been spontaneously produced from the hybridoma, by the following method.

(c)  Test for TRF Activity

Spleen cells of a BALB/C mouse immunized with 100 μg of dinitrophenylated keyhole limpet hemocyanin (DNP-KLH) was suspended and treated with the anti-Thy 1.2 antibody and complement to remove the T cells.  To this was added 80 ng/ml of dinitrophenylate ovalbumin (DNP-OVA) as the antigen, and the cell concentration was adjusted to $0.75 \times 10^6$ cells/100 μl, after which this was inoculated to the respective wells of a micro-tissue culture plate.  At the same time, 100 μl of each specimen to be tested for the TRF activity was added to each well.  On the 5th day from the start of culture, the cells were individually recovered from each well, and the number of the antibody producing cells appeared was judged by Jerme's plaque method or Cunningham method both well known in the art.  The higher the TRF activity of the specimen added, the greater the number of the antibody producing cells.

As the result of the test on the TRF activity in each culture supernatant from the obtained 170 kinds of hybridomas, C262-J16 was obtained as the hybridoma which spontaneously produces TRF as shown in Table 1.

(d)  Culture of TRF Producing Hybridoma C262-J16

The obtained C262-J16 was suspended in a 10% fetal bovine serum containing RPMI-1640 medium at an initial concentration of $0.2 \times 10^5$ cells/ml, then one of the following cultures:

A:  static culture in a scale of 10 ml    medium thickness of 1.3 mm) in a plastic Petri dish of 10 cm in diameter;

B:  static culture in a scale of 25 ml    medium thickness of 2.0 mm) in a plastic Petri dish having a bottom surface area when culturing of 125 $cm^2$;

C:  static culture in a scale of 100 ml (the medium thickness of 8.0 mm) in a plastic Petri dish having a bottom surface area when culturing of 125 $cm^2$;

D:  shaking culture using the flask under the conditions the same as in C while shaking at 10 rpm;

E:  static culture in a scale of 1 liter in a roller bottle of 10 cm in diameter and 850 $cm^2$ in circumferential surface area;

F: rotatory culture using the roller bottle under the conditions the same as in E;

G:  stirred culture in a scale of 1 liter in a spinner flask for one liter culture; or

H:  stirred culture with aeration with 5 % $CO_2$ containing air in a scale of 50 liters in a cell culture tank for 50 liter culture;

were conducted, and at 24 hours' intervals, a one-ml sample of each suspension was taken from the incubator to measure the the cell concentration.

Table 2 shows the results of the cell proliferation in the culture modes A, B, C and D.  Where the medium was thin as in A and B, the oxygen supply was sufficient and C262-J12 proliferated about 15 times in 3 - 4 days, whereas the medium was thick as in C, the oxygen dissolved in the medium was inadequate and the hybridoma did not proliferate at all. However, even when the medium was thick, when the oxygen supply to the medium was increased by agitating the medium by shaking the flask as in D, the hybridoma had proliferated about 15 times in 3 - 4 days.

Table 3 shows the cell proliferation in the culture modes E and F. When the roller bottle was employed for steadily supplying fresh oxygen to the medium and rotatory culture was conducted as in F, the hybridoma exhibited favorable proliferation. On the contrary, in E where culture was conducted without rotating the roller bottle, the cells did not proliferate at all.

Table 4 shows the cell proliferation in the culture modes G and H. When a single mass-culture is needed, the oxygen supply to the medium may be ensured by conducting a stirred culture. In stirred culture both in one-liter and 50 liter scales, the hybridoma proliferated favorably and reached about 10 times in 3 - 4 days.

(e)  Production of TRF with TRF Producing Hybridoma C262-J16.

The C262-J16 hybridoma was cultured by the method A - H described in (d), and on the 4th day of the culture, the culture supernatant was taken and the produced TRF activity was measured according to the method for testing the TRF activity described in (c).

As set forth in Table 5, in any culture mode of Petri dish

- 21 -

culture, flask culture, shaken culture, rotatory culture and stirred culture, TRF is produced in the culture supernatant as long as the cell proliferation proceeds favorably (A, B, D, F, G and H). Therefore, by employing these methods, TRF may be easily mass-produced by mass-culture of the TRF producing hybridoma. On the other hand, when the cells do not proliferate at all, TRF is consequently not produced in the medium.

Example 2

Preparation of T Lymphocyte Hybridoma Which Spontaneously Produces IL 2 and Mass-production of IL 2 by its Mass-culture

(a)  Preparation of Hybridoma

According to the method in (a) of Example 1, hybridomas were prepared, and 326 kinds of hybridoma clones were obtained.

(b)  Selection of IL 2 Producing Hybridoma Clone

The obtained hybridomas were each cultured according to the method in (b) of Example 1, and each culture supernatant was collected. This culture supernatant was tested for the IL 2 activity according to the following method, to judge whether IL 2 had been spontaneously produced.

(c)  Test for IL 2 Activity

A specimen to be tested for the IL 2 activity was diluted within an appropriate concentration range and allotted 100 µl each to the respective wells. At the same time, activated T lymphocytes prepared by the method taught in Nature, 268, 154 (1977) were adjusted to a concentration of $4 \times 10^3$ cells/100 µl and alloted 100 µl each to the respective wells. Twenty-four to forty-eight hours later, 0.5 µCi of tritium-labeled thymidine was added and a few hours later, the cells were harvested in conventional manner well known in the art, and the radioactivity uptake by the cells was measured. Since the tritium-labeled thymidine uptake by the activated T lymphocytes is increased with the increase in the IL 2 activity in the culture supernatant, the clone producing IL 2 in the culture supernatant may be easily selected.

As the result of the test on the culture supernatants of the obtained 326 kinds of hybridomas, as shown in Table 6, the clone hybridoma/S30-M310 which spontaneously produces IL 2 was obtained.

(d)  Culture of IL 2 Producing Hybridoma S30-M301

The obtained S30-M310 was suspended in a 10% fetal bovine serum containing RPMI-1640 medium at an initial concentration of $0.2 \times 10^5$ cells/ml, and the following cultures were conducted: static culture according to C in (d) of Example 1, rotatory culture according to F and tank culture according to H.  The cell suspension was taken from the incubator at 24 hours' intervals, to measure the cell concentration.

Table 7 shows the results of the cell proliferation in the various culture modes.  S30-M301, like C262-J16, did not proliferate when the medium was thick, and proliferation was made possible by replenishing the deficiency of the dissolved oxygen by conducting rotatory culture or stirred culture.  Also with S30-M 301, roller culture in a one-liter scale and tank culture in a 50-liter scale were possible.

(e)  Production of IL 2 with IL 2 Producing Hybridoma S30-M301

The S30-M301 hybridoma was cultured using the methods C, F and H in (d) of Example 1, and each culture supernatant was collected on the third day and fourth day of culture, to measure the produced IL 2 activity according to the IL 2 activity testing method described in (c).

As set forth in Table 8, IL 2 is produced in the culture supernatant when conducting either rotatory culture in a one-liter scale or tank stirred culture in a 50-liter scale. Therefore, by employing these methods, IL 2 may be easily mass-produced by mass-culture of the IL 2 producing hybridoma.

Example 3

Preparation of T Lymphocyte Hybridoma Which Spontaneously Produces MAF and Mass-production of MAF by its Mass-culture

(a) Production of Hybridoma

According to the method in (a) of Example 1, hybridomas were prepared, and 326 kinds of hybridoma clones were obtained.

(b) Selection of MAF Producing Hybridoma Clone

The obtained hybridomas were each cultured according to the method in (b) of Example 1, and each culture supernatant was collected. This culture supernatant was tested for the MAF activity according to the following method, to judge whether MAF had been spontaneously produced.

(c) Test for MAF Activity

A commercially available ICR mouse was intraperitoneally administered with one ml of physiological saline containing 10% by weight of proteose peptone, and 4 days later, the peritoneal exudate cells were collected in conventional manner well known in the art. The cells were adjusted to $8 \times 10^5$ cells/ml

and allotted 1 ml each to the respective wells on a 24-welled tissue culture plate, followed by culture for 3 hours. The unattched cells were washed away with the medium to leave only the attached cells, and 1 ml of a sample to be measured for the MAF activity was added. After 24 hours of culture, the sample was removed, the well was washed once with the medium, and 100 $\mu$l of a suspension of P815 cells (cells generally employed in the technical field of the invention) adjusted to $2 \times 10^5$ cells/ml was added. After 20 hours of culture, 1 $\mu$Ci of tritium-labeled thymidine was added and culture was continued for further 4 hours, after which the cells were harvested in a conventional manner well known in the art, and the radioactivity uptake by the P815 cells was measured. Since the attached cells activated by MAF (phagocytes) inhibit the DNA synthesis by P815, the presence of the MAF activity in the hybridoma culture supernatant may be easily judged.

As the result of the test on the culture supernatants of the obtained 326 hybridomas, as shown in Table 9, the hybridoma clone A31-F29 which spontaneously produces MAF was obtained.

(d) Culture of MAF Producing Hybridoma A31-F29

The obtained A31-F29 was suspended in a 10% fetal bovine serum containing RPMI-1640 medium at an initial concentration of $0.2 \times 10^5$ cells/ml, and the following cultures were conducted: static culture according to C in (d) of Example 1 and stirred culture according to H. The cell suspension was taken from the incubator at 24 hours' intervals, to measure the cell concentration.

Table 10 shows the results of the cell proliferation in the various culture modes. Like the other hybridomas hereinabove described, when the medium was thick, A31-F29 did not proliferate in static culture, but it exhibited proliferation when a tank stirred culture was conducted.

(e) Production of MAF with MAF Producing Hybridoma A31-F29

A31-F29 was cultured according to the methods C and H in (d) of Example 1, then on the third day and fourth day, each culture supernatant was collected and the produced MAF activity was measured according to the MAF activity testing method described in (c).

As set forth in Table 11, MAF was produced in the culture supernatant when tank culture in a 50-liter scale was conducted. Therefore, MAF may be easily mass-produced by mass-culture of the MAF producing hybridoma.

Example 4

Preparation of T Lymphocyte Hybridoma Which Spontaneously Produces $\gamma$-Interferon and Mass-production of $\gamma$-Interferon by its Mass-culture

(a) Preparation of Hybridoma

Spleen cells of a C57BL/6 mouse (commercially available) were suspended in a 10% fetal bovine serum containing RPMI-1640 medium, adjusted to a concentration of $3 \times 10^6$ cells/ml, and, after adding phytohemaggulutinin (PHA) at a concentration of 3 µg/ml, cultured in a 24-welled tissue culture plate for 24 hours. After the culture, the cells were collected, the B cells, immature thymus gland cells, phagocytes and dead cells were removed as in Example 1, to prepare a cell suspension having an increased concentration of the T cells. The cells were fused with BW5147·AZG$^R$·OU$^R$ in a manner similar to those in Example 1 to obtain 16,723 kinds of hybridoma clones.

(b) Selection of $\gamma$-Interferon Producing Hybridoma Clone

The obtained hybridomas were each cultured according to the method in (b) of Example 1, and each culture supernatant was collected. This culture supernatant was tested for the IFN (hereinafter sometimes referred to as IFN) activity according to the following method, to judge whether $\gamma$-interferon (hereinafter sometimes referred to as $\gamma$-IFN) had been spontaneously produced.

(c) Test for Interferon Activity

Mouse L cells at a concentration of 5.5 x $10^5$ cells/ml were allotted 100 $\mu$l each to the respective wells on a 96-welled tissue culture plate. After culturing 6 - 12 hours, 50 $\mu$l of each sample to be measured for the interferon activity was added to each well, and cultured for further 8 - 20 hours. Thereafter, the medium was removed, and 100 $\mu$l of vesicular stomatitis virus (VSV) adjusted to $10^5$ - $10^6$ colony-forming units/100 $\mu$l was inoculated to each well. After culturing 1 - 2 days, the medium was removed, 50 $\mu$l of a 0.02% neutral red dye solution was added to each well, and culture was effected for 30 minutes. Then, each well was washed with phosphate buffered physiological saline (pH 7.2) twice and the virus was inactivated by irradiating with ultraviolet light. 100 $\mu$l of 0.001 N hydrochloric acid containing 20 - 30% of ethanol was added to each well to dissolve the dye and the absorbance (545 nm) was measured.

The IFN titer is determined as follows: Two-fold serial dilutions of the specimen are prepared and each activity is measured, after which the dilution (common logarithms) at which 50 % of the dye is absorbed by the normal cells is determined and its reciprocal number is designated as the IFN titer.

As the result of the test on the culture supernatants of the obtained 16,723 hybridomas, as shown in Table 12, the hybridoma Fl3-K15-Il26 which spontaneously produces IFN was obtained. Since the IFN produced by this hybridoma was inactivated at pH 2, this was found to be $\gamma$-IFN.

(d) Culture of $\gamma$-IFN Producing Hybridoma Fl3-K15-Il26

The obtained Fl3-K15-Il26 was suspended in a 10% fetal bovine serum containing RPMI-1640 medium at an initial concentration of 0.2 x $10^5$ cells/ml, and the following cultures were conducted: static culture according to C in (d) of Example 1 and rotatory culture according to F. The cell suspension was taken from the incubator at 24 hours' intervals, to measure the cell concentration.

Table 13 shows the results of the cell proliferation in the two culture modes. Fl3-K15-Il26 does not proliferate in static culture because of the deficiency of oxygen, but it proliferates when rotatory culture is conducted.

(e) Mass-production of $\gamma$-IFN with $\gamma$-IFN Producing Hybridoma Fl3-K15-Il26

Fl3-K15-Il26 was cultured according to the methods C and F in (d) of Example 1, then on the third day and fourth day, each culture supernatant was collected and the produced $\gamma$-IFN activity was measured according to the $\gamma$-IFN activity testing method described in (c).

Table 14 shows the results thereof, from which it can be seen that when the hybridoma favorably proliferates by rotatory culture, $\gamma$-IFN is produced in the culture supernatant, whereas in static culture, the hybridoma does not proliferate at all and consequently no $\gamma$-IFN is produced.

Example 5

Preparation of T Lymphocyte Hybridoma Which Spontaneously Produces CSF and Mass-production of CSF by its Mass-culture

(a) Preparation of Hybridoma

According to the method in (a) of Example 1, hybridomas were prepared, and 504 kinds of hybridoma clones were obtained.

(b) Selection of CSF Producing Hybridoma Clone

The obtained hybridomas were each cultured according to the method in (b) of Example 1, and each culture supernatant was collected. This culture supernatant was tested for the CSF activity according to the following method, to judge whether CSF had been spontaneously produced.

(c) Test for CSF Activity

0.2 ml of the culture supernatant of the hybridoma to be tested for the CSF activity, 0.2 ml of horse serum, 0.4 ml of 2.2% methyl cellulose and 0.2 ml of a mouse bone marrow cell suspension at $5 \times 10^5$ cells/ml were prepared in a medium of $\gamma$-modified Eagle medium ($\gamma$-MEM), added to a Petri dish of 3 cm in diameter, and      culture      was effected for 7 days. After the culture, the number of colonies formed by the bone marrow cells was measured un-

der a microscope. Since the higher the CSF activity in the culture supernatant of the hybridoma, the higher the colony number, the clone producing CSF in the culture supernatant may be easily selected.

As the result of the test on the culture supernatants of the obtained 504 hybridomas, as shown in Table 15, the hybridoma clone K5-P33 which spontaneously produces CSF was obtained.

(d)    Culture of CSF Producing Hybridoma K5-P33

The obtained K5-P33 was suspended in a 10% fetal bovine serum containing RPMI-1640 medium at an initial concentration of 0.2 x $10^5$ cells/ml, and the following cultures were conducted: static culture, rotary culture and tank culture according to C, F and H in (d) of Example 1, respectively. The cell suspension was taken from the incubator at 24 hours' intervals, to measure the cell concentration.

Table 16 shows the results of the cell proliferation in the various culture modes. Like the other hybridomas hereinbefore described, K5-P33 did not proliferate when the medium was thick, but by improving the deficiency of dissolved oxygen by conducting rotatory  culture or stirred culture, it proliferated.

Also with this hybridoma, mass-culture in a scale of 1 - 50 liters is possible.

(e)    Production of CSF with CSF Producing Hybridoma K5-P33

K5-P33 was cultured according to the methods C, F and H

in (d) of Example 1, then on the fourth day, each culture supernatant was collected and the produced CSF activity was measured according to the CSF activity testing method described in (c).

As set forth in Table 17, CSF is produced in the culture supernatant when conducting either rotatory culture in a one-liter scale or tank culture in a 50-liter scale. Therefore, by employing these methods, CSF may be easily mass-produced by mass-culture of the CSF producing hybridoma.

Example 6

Production of Human IL 2 Producing Hybridoma

(a) Production of Hybridoma

Peripheral blood of a tuberculin-positive human was sampled and a lymphocyte suspension was prepared in conventional manner well known in the art. The cells were suspended in a medium (RPMI-1640 + 10% fetal bovine serum) at a concentration of 2 x $10^6$ cells/ml, and, after adding 25 µg/ml of PPD, allotted 1 ml each to the respective wells on a 24-welled tissue culture plate, followed by culture at 37°C in 5% $CO_2$ for 24 - 48 hours. After the culture, the lymphocytes were recovered from the plate and treated with the anti-human immunoglobulin antibody and complement to remove the B cells. The thus obtained cell suspension having an increased concentration of the T cells was fused with BW5147·AZG$^R$·OU$^R$ similarly as in (a) of Example 1, to obtain 566 kinds of hybridoma clones.

(b) Selection of IL 2 Producing Hybridoma

The obtained hybridoma was adjusted to a cell concentration of 0.2 x $10^5$ cells/ml, and 5 ml thereof was cultured in a plastic Petri dish of 6 cm in diameter. On the fourth day, the culture supernatant was taken. This culture supernatant was tested for the IL 2 activity according to the following method, to judge whether IL 2 had been spontaneously produced.

(c) Test for IL 2 Activity

Two-fold serial dilutions of the specimen to be tested were prepared and 100 µl of each dilution was allotted to the wells on a tissue culture plate. 100 µl of activated lympho-cytes prepared according to the method taught in Nature, 268, 154 (1977) at a concentration of $4 \times 10^3$ cells/ml was added to each well. 24 - 48 hours later, 0.5 µCi of tritium-labeled thymidine was added and a few hours later, the cells were harvested in conventional manner well known in the art, and the radioactivity uptake by the cells was measured.

The IL 2 titer is determined as the reciprocal number of the dilution which exhibits 50% radioactivity uptake relative to the maximum radioactivity uptake exhibited by the T lymphocytes, and is expressed in units.

As the result of the test on the culture supernatants of the 566 hybridomas for IL 2 activity, as.shown in Table 18, the hybridoma clones HM7-B191 and HR-A25 which spontaneously produce human IL 2 were obtained.

The IL 2 produced by these hybridomas were examined for the properties by salting out with ammonium sulfate, gel permeation on Sephacryl S-200 and isoelectric electrophoresis, and as the result, it had a molecular weight of about 15,000, an isoelectric point of 6.5 and did not lose activity in a pH range of 2.0 - 9.0, thus exhibiting properties similar to those of human T cell-derived IL 2.

Table 1

| TRF sample | | antibody forming cells per $1 \times 10^6$ cells |
|---|---|---|
| medium | | 23 |
| culture supernatant of BW5147 | | 18 |
| mouse T cell derived TRF | | 191 |
| culture supernatant of hybridoma | A151-A12 | 12 |
| | A281-B36 | 67 |
| | C260-H20 | 42 |
| | C262-J16 | 188 |
| | C278-J16 | 146 |
| | D107-M31 | 18 |
| | D109-K28 | 31 |

Table 2

| | | culture time (day) | | | |
|---|---|---|---|---|---|
| | | 1 | 2 | 3 | 4 |
| A | cell density* | 0.9 | 2.6 | 7.1 | 14.5 |
| | viability** | 93 | 92 | 98 | 95 |
| B | cell density* | 0.9 | 2.8 | 7.4 | 15.1 |
| | viability** | 91 | 96 | 95 | 98 |
| C | cell density* | 0.3 | 0.1 | 0 | 0 |
| | viability** | 70 | 31 | 0 | 0 |
| D | cell density* | 0.7 | 2.4 | 6.0 | 14.0 |
| | viability** | 90 | 91 | 94 | 91 |

$* \times 10^5$ cells/ml, ** %

Table 3

|  |  | culture time (day) | | | |
|---|---|---|---|---|---|
|  |  | 1 | 2 | 3 | 4 |
| A | cell density* | 0.9 | 2.6 | 7.1 | 14.5 |
|  | viability** | 93 | 92 | 98 | 95 |
| E | cell density* | 0.1 | 0.05 | 0 | 0 |
|  | viability** | 56 | 23 | 1 | 0 |
| F | cell density* | 1.1 | 2.8 | 7.5 | 17.9 |
|  | viability** | 97 | 99 | 100 | 96 |

$* \times 10^5$ cell/ml , ** %

Table 4

|  |  | culture time (day) | | | |
|---|---|---|---|---|---|
|  |  | 1 | 2 | 3 | 4 |
| A | cell density* | 0.9 | 2.6 | 7.1 | 14.5 |
|  | viability** | 93 | 92 | 98 | 95 |
| G | cell density* | 0.7 | 4.6 | 7.5 | 13.8 |
|  | viability** | 93 | 90 | 88 | 90 |
| H | cell density* | 1.0 | 4.2 | 7.3 | 14.1 |
|  | viability** | 96 | 99 | 91 | 91 |

$* \times 10^5$ cell/ml , ** %

Table 5

| TRF sample | | antibody forming cells per $1 \times 10^6$ cells |
|---|---|---|
| medium | | 5 |
| mouse T cell derived TRF | | 248 |
| culture supernatant of hybridoma | A | 208 |
| | B | 220 |
| | C | 3 |
| | D | 174 |
| | E | 0 |
| | F | 249 |
| | G | 197 |
| | H | 255 |

## Table 6

| IL 2 sample | | IL 2 (unit/ml) |
|---|---|---|
| medium | | 0 |
| culture supernatant of BW5147 | | 0 |
| mouse T cell derived IL 2 | | 150 |
| culture supernatant of hybridoma | S15-B140 | 0 |
| | S17-B153 | 32 |
| | S17-B180 | 2 |
| | S18-C280 | 0 |
| | S25-A750 | 0 |
| | S29-A831 | 8 |
| | S30-M301 | 120 |
| | S30-P305 | 190 |

## Table 7

| | | culture time (day) | | | |
|---|---|---|---|---|---|
| | | 1 | 2 | 3 | 4 |
| C | cell density* | 0.7 | 0.7 | 0.3 | 0 |
| | viability** | 54 | 42 | 21 | 3 |
| F | cell density* | 1.0 | 4.1 | 8.2 | 14.6 |
| | viability** | 92 | 97 | 99 | 98 |
| H | cell density* | 0.8 | 3.9 | 6.3 | 13.8 |
| | viability** | 90 | 91 | 93 | 92 |

$* \times 10^5$ cells/ml , ** %

Table 8

| IL2 sample | | IL2 (unit/ml) | |
|---|---|---|---|
| | | day 3 | day 4 |
| medium | | 0 | - |
| mouse T cell derived IL2 | | .150 | - |
| culture supernatant of hybridoma | C | 0 | 0 |
| | F | 95 | 177 |
| | H | 60 | 125 |

Table 9

| MAF sample | | MAF activity* |
|---|---|---|
| medium | | 0 |
| culture supernatant of BW5147 | | 3 |
| mouse T cell derived MAF | | 68 |
| culture supernatant of hybridoma | A31-F29 | 60 |
| | A34-F31 | 7 |
| | B33-F28 | 1 |
| | B35-F54 | 21 |
| | B35-F66 | 13 |
| | B35-F67 | 25 |
| | B35-F71 | 18 |
| | B35-F73 | 3 |

\* % inhibition of DNA synthesis of P815 target cells.

## Table 10

| | | culture time (day) | | | |
|---|---|---|---|---|---|
| | | 1 | 2 | 3 | 4 |
| C | cell density* | 0.4 | 0.05 | 0.05 | 0 |
| | viability** | 72 | 12 | 1 | 0 |
| H | cell density* | 0.7 | 2.5 | 7.6 | 16.6 |
| | viability** | 98 | 98 | 99 | 94 |

$* \times 10^5$ cells/ml , ** %

## Table 11

| MAF sample | | MAF activity* | |
|---|---|---|---|
| | | day 3 | day 4 |
| medium | | 4 | – |
| mouse T cell derived MAF | | 66 | – |
| culture supernatant of hybridoma | C | 7 | 5 |
| | H | 39 | 53 |

* % inhibition of DNA synthesis of P815 target cells

Table 12

| ƒ-Interferon sample | | Interferon (unit/ml) |
|---|---|---|
| medium | | 16 |
| culture supernatant of BW5147 | | 0 |
| mouse ƒ-Interferon | | 1100 |
| culture supernatant of hybridoma | A10-K15-F16 | 20 |
| | A16-K15-D27 | 150 |
| | D7-K15-K112 | 0 |
| | F13-K15-I126 | 600 |
| | F13-K15-I181 | 1 |
| | G15-K15-A27 | 0 |
| | G12-K15-B33 | 1 |
| | H13-K15-J27 | 8 |

Table 13

| | | culture time (day) | | | |
|---|---|---|---|---|---|
| | | 1 | 2 | 3 | 4 |
| C | cell density* | 0.2 | 0.1 | 0.05 | 0.05 |
| | viability** | 72 | 41 | 3 | 1 |
| F | cell density* | 1.0 | 3.8 | 7.3 | 9.0 |
| | viability** | 88 | 86 | 91 | 82 |

$* \times 10^5$ cells/ml , ** %

0077571

Table 14

| $\gamma$ -Interferon sample | | Interferon (units/ml) | |
|---|---|---|---|
| | | day 3 | day 4 |
| medium | | 12 | − |
| mouse $\gamma$-Interferon | | 1100 | − |
| culture supernatant of hybridoma | C | 2 | 2 |
| | F | 480 | 620 |

Table 15

| CSF sample | | colonies /$10^6$ cells |
|---|---|---|
| medium | | 15 |
| culture supernatant of BW5147 | | 23 |
| mouse T cell derived CSF | | 261 |
| culture supernatant of hybridoma | K1-P2 | 53 |
| | K1-P32 | 7 |
| | K2-P5 | 122 |
| | K4-P78 | 17 |
| | K5-P33 | 306 |
| | K5-P46 | 81 |
| | K6-P71 | 224 |
| | K7-P28 | 25 |

Table 16

| | | culture time (day) | | | |
|---|---|---|---|---|---|
| | | 1 | 2 | 3 | 4 |
| C | cell density* | 0.7 | 1.0 | 0.8 | 0.3 |
| | viability** | 82 | 77 | 61 | 44 |
| F | cell density* | 2.1 | 4.3 | 9.0 | 18.7 |
| | viability** | 98 | 99 | 100 | 98 |
| H | cell density* | 2.1 | 4.4 | 11.8 | 16.5 |
| | viability** | 94 | 99 | 99 | 96 |

$* \times 10^5$ cells/ml , ** %

Table 17

| CSF sample | | colonies/$10^6$ cells |
|---|---|---|
| medium | | 19 |
| mouse T cell derived CSF | | 292 |
| culture supernatant of K5-P33 | C | 4 |
| | F | 197 |
| | H | 211 |

Table 18

0077571

| IL2 sample | | IL2 (units/ml) |
|---|---|---|
| medium | | 0 |
| human T cell derived IL2 | | 95 |
| culture supernatant of BW5147 | | 0 |
| culture supernatent of hybridoma | HM7-B191 | 116 |
| | HM7-B233 | 0 |
| | HM7-B245 | 0 |
| | HM7-B253 | 0 |
| | HR-A11 | 0 |
| | HR-A25 | 86 |
| | HR-A38 | 0 |
| | HR-A42 | 0 |
| | HR-A58 | 0 |

## CLAIMS

1. A process for producing a lymphokine in purified form or as a lymphokine containing culture supernatant, characterized by the following process steps :

(1) collecting lymphocytes from an animal or a human sensitized by an immunogen which induces the production of an antibody,

(2) selectively concentrating the lymphokine producing cells,

(3) forming a fused cell hybrid between the lymphokine producing cells and animal derived tumor cells,

(4) cloning the obtained hybrid,

(5) selecting the clone which spontaneously produces the lymphokine,

(6) culturing the clone to produce the lymphokine, and optionally

(7) recovering the lymphokine from the culture and purifying the lymphokine.

2. A process acccording to claim 1, wherein lymphocytes of human origin or from a mouse or rat are used.

3. The process according to claim 1 or 2, wherein the immunogen are living cells or dead cells of Mycobacterium tuberculosis.

4. A process for producing a lymphokine which is characterized by culturing and proliferating a T lymphocyte-derived hybridoma which spontaneously produces a lymphokine either with or without aeration under such culturing that an adequate amount of oxygen is supplied to the cells by enhancing the amount of oxygen dissolved in the medium by shaking, stirring, rotating or the like to produce and accumulate the lymphokine in the medium and optionally recovering it.

5. The process according to any of claims 1 to 4, wherein the T lymphocyte-derived hybridoma is capable of producing interleukin 2, colony stimulating factor, T cell replacing factor, macrophage activating factor or immune interferon.

6. A lymphokine in purified form or in the form of a lymphokine containing culture supernatant, obtainable by the following process :

  (1) collecting lymphocytes from an animal or a human sensitized by an immunogen which induces the production of an antibody,

  (2) selectively concentrating the lymphokine producing cells,

(3) forming a fused cell hybrid between the lymphokine producing cells and animal derived tumor
cells,

(4) cloning the obtained hybrid,

(5) selecting the clone which spontaneously produces
the lymphokine,

(6) culturing the clone to produce the lymphokine, and
optionally

(7) recovering the lymphokine from the culture and
purifying the lymphokine.


7. A product according to claim 6, wherein the lympho-
line is interleukin 2, colony stimulating factor, T
lymphocyte replacing factor, macrophage activating factor
and/or immune interferon.